(19) Europäisches Patentamt
European Patent Office
Office européen des brevets

(11) Numéro de publication: **0 003 445**
**B1**

(12) # FASCICULE DE BREVET EUROPEEN

(45) Date de publication du fascicule de brevet: 25.11.81

(51) Int. Cl.[3]: C 07 D 453/02, A 61 K 31/435

(21) Numéro de dépôt: 79400001.8

(22) Date de dépôt: 02.01.79

(54) **Dérivés d'aza-1 bicyclo(2,2,2)octane, leur procédé de préparation et produits intermédiaires, et médicaments les contenant.**

(30) Priorité: 10.01.78 FR 7800490

(43) Date de publication de la demande:
08.08.79 Bulletin 79/16

(45) Mention de la délivrance du brevet:
25.11.81 Bulletin 81/47

(84) Etats Contractants Désignés:
BE CH DE FR GB LU NL SE

(56) Documents cités:
FR - A - 1 235 918

JOURNAL OF THE ORGANIC CHEMISTRY *22*, November 1957, Columbus Ohio

T. D. PERRINE: "Quinuclistines 1,4-Phenylquinuclidines as potential analgesics" pages 1484—89.

(73) Titulaire: **PHARMINDUSTRIE**
**35 Quai du Moulin de Cage**
**F-92231 Gennevilliers (FR)**

(72) Inventeur: **Gueremy, Claude Georges Alexandre**
**3 rue Daumesnil**
**F-78800 Houilles (FR)**
Inventeur: **Hodac, Françoise**
**182 bis Boulevard Pereire**
**F-75017 Paris (FR)**
Inventeur: **Renault, Christian Louis Albert**
**10, Allée Réaumur**
**F-95150 Taverny (FR)**

(74) Mandataire: **Houssin, Jean Produits Chimiques Ugine Kuhlmann et al,**
**Service Propriété Industrielle Tour Manhattan**
**Cedex 21**
**F-92087 Paris la Défense (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 003 445 B1

Dérivés d'aza-1 bicyclo [2,2,2] octane, leur procédé de préparation, et produits intermédiaires et médicaments les contenant

L'invention concerne de nouveaux dérivés d'aza-1 bicyclo [2,2,2] octane, utiles notamment comme psychotropes, plus particulièrement ent tant qu'agents antidépresseurs et antiparkinsoniens.

Il est déjà connu, par le brevet FR—A—1 235 918 et le document T. D. PERRINE, J. Org. Chem., *22*, 1484—1489, (1957), des aryl-3 ou phényl-4 quinuclidines ayant un effet stimulant sur le système nerveux central et le centre respiratoire ou un effet analgésique.

Les dérivés selon l'invention peuvent être représentés par la formule:

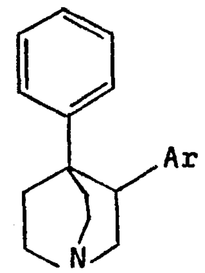

(I)

dans laquelle Ar représente un groupe phényle, éventuellement substitué par un atome d'halogène ou par un groupe trifluorométhyle.

Les produits de formule (I) peuvent être préparés par hydrogénation catalytique des produits de formule (II):

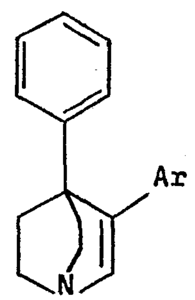

(II)

dans laquelle Ar a la même signification que dans la formule (I).

Cette réaction est effectuée au sein d'un solvant inerte, par exemple un alcool comme le méthanol ou l'éthanol ou un acide comme l'acide acétique, le produit à hydrogéner pouvant être utilisé sous forme de base libre ou sous forme d'un sel de la base.

Le catalyseur utilisé est le palladium, le nickel, le rhodium, le ruthénium ou le platine ou un oxyde de ces trois dernier métaux. La réaction est effectuée sous atmosphère d'hydrogène, à une température qui peut varier entre 20°C et 120°C.

Une méthode particulièrement bonne pour effectuer l'hydrogénation catalytique des composés de formule (II) consiste à opérer dans le méthanol, en présence de charbon palladié, sous une pression d'hydrogène de 20 à 50 bars et à une température d'environ 50°C.

Une fois l'hydrogénation terminée le mélange réactionnel est traité suivant des méthodes classiques, physiques (évaporation, extraction par un solvant, distillation, cristallisation, chromatographie, etc...) ou chimiques (formation de sel et régénération de la base etc...) afin d'isoler les produits de formule (I) à l'état pur.

Les produits de formule (II) peuvent être préparés par déshydratation des produits de formule (III):

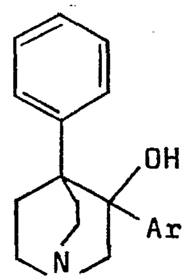

(III)

dans laquelle Ar a la même signification que dans la formule (I).

Cette réaction est effectuée au moyen d'un agent déshydratant appropié, en présence ou en

l'absence d'un solvant inerte, suivant des méthodes connues en soi, par exemple celles décrites par WAGNER et ZOOK (Synthetic Organic Chemistry, page 32, J. Wiley, 1965). Elle est avantageusement réalisée par chauffage au reflux en présence d'un chlorure d'acide tel que le chlorure de thionyle comme agent déshydratant.

Une fois la réaction terminée, le mélange réactionnel est traité suivant des méthodes classiques (évaporation, traitement du résidu à l'eau et extraction à l'aide d'un solvant, etc...) afin d'isoler les produits de formule (II) à l'état pur, soit sous forme de la base soit souse forme d'un sel de la base.

Les produits de formule (III) peuvent être préparés par action sur la phényl-4 quinuclidinone-3[décrite par T. D. PERRINE J. Org. Chem. *22* 1484 (1957)] d'un dérivé organométallique de formule Ar—M, dans laquelle M représente soit un atome de lithium soit un groupe Mg—X (X étant un atome d'halogène, généralement brome ou iode) et Ar a la même signification que dans la formule (I).

On réalise cette réaction au sein d'un solvant inerte tel qu'un éther-oxyde (éther éthylique ou tétrahydrofuranne), éventuellement mélangé avec d'autres solvants tels que l'hexaméthylphosphotriamide ou un hydrocarbure. La réaction est effectuée à une température comprise entre 0°C et la température d'ébullition du milieu utilisé. Le mélange réactionnel est ensuite traite à l'eau, à basse température et en présence d'un acide, et le composé de formule (III) formé est isolé par des méthodes classiques.

Les composés de formules (II) et (III) sont nouveaux et font partie en tant que tels de l'invention.

Les composés de formule (I) à l'état de base libre peuvent être transformés en sels d'addition avec un acide minéral ou organique par action d'un tel acide au sein d'un solvant approprié.

Les exemples suivants illustrent l'invention sans la limiter. Les exemples 1 et 2 concernent la préparation des composés de formule (III), l'exemple 3 celle des composés de formule (II) et l'exemple 4 celle des composés de formule (I).

## Exemple 1

Diphenyl-3,4 aza-1 bicyclo[2,2,2]octanol-3

A 250 ml d'une solution 2,4 M de bromure de phényl-magnésium dans le tétrahydrofuranne on ajoute, à la température ambiante, une solution de 30 g de phényl-4 aza-1 bicyclo[2,2,2]octanone-3 dans 500 ml de tétrahydrofuranne. On maintient le milieu réactionnel 20 heures à 50°C puis on le verse sur 2,7 kg de glace contenant 150 ml d'acide chlorhydrique concentré. On décante et distille la phase organique sous pression réduite pour éliminer le tétrahydrofuranne, ce qui entraîne la cristallisation du chlorhydrate de diphényl-3,4 aza-1 bicyclo[2,2,2]octanol-3. Après lavage à l'eau et séchage, on obtient 17,8 g de ce produit, dont le poit de fusion est supérieur à 250°C.

La structure du produit obtenu est comformée par son spectre infra-rouge (I.R.), qui présente une bande d'absorption à 3250 $cm^{-1}$ correspondant au groupe OH.

## Exemple 2

Diphenyl-3,4 aza-1 bicyclo[2,2,2]octanol-3

A une solution de 12 g de phényl-4 aza-1 bicyclo[2,2,2] octanone-3 dans 360 ml de tétrahydrofuranne anhydre on ajoute, à 0°C et en 4 heures, 95 ml d'une solution 1,9 M de phényllithium dans un mélange benzène-éther.

On maintient 1 heure à 0°C puis on verse le mélange réactionnel sur 300 g de glace et 20 ml d'acide chlorhydrique concentré. On élimine le tétrahydrofuranne par distillation sous pression réduite et ajoute du chlorure de sodium à la suspension aqueuse. On filtre et lave le précipité à l'eau, à l'éther et à l'acétone. Après séchage on obtient 12,2 g de chlorhydrate de diphényl-3,4 aza-1 bicyclo[2,2,2]-octanol-3, dont le point de fusion est supérieur à 250°C. La structure du produit obtenu est confirmée par son spectre I.R., qui présente un bande d'absorption à 3250 $cm^{-1}$ correspondant au groupe OH.

## Exemple 3

Diphenyl-3,4 aza-1 bicyclo[2,2,2]octene-2

On porte 3 heures à reflux une solution de 16,8 g de chlorhydrate de diphényl-3,4 aza-1 bicyclo[2,2,2]octanol-3 dans 425 ml de chlorure de thionyle. Puis on concentre la solution sous pression réduite et on fait précipiter le composé formé par addition de toluène. Après lavage à l'éther et séchage du précipité, on obtient 15,7 g de chlorhydrate de diphényl-3,4 aza-1 bicyclo[2,2,2]octéne-2 fondant à 245°C.

Analyse pour: $C_{19}H_{19}N$, HCl

| | C | H | N |
|---|---|---|---|
| Calculé | 76,7 | 6,72 | 4,71 |
| Trouvé | 76,8 | 6,9 | 4,7 |

Exemple 4

Diphenyl-3,4 aza-1 bicyclo[2,2,2]octane

On hydrogène pendant 7 heures, à 50°C et sous une pression d'hydrogène de 30 bars. 7,4 g de chlorhydrate de diphényl-3,4 aza-1 bicyclo[2,2,2]octène-2 en solution dans 75 ml de méthanol, en présence de 1,8 g de charbon palladié. Après filtration puis élimination du solvent sous pression réduite, on récupère 7 g de produit que l'on recristallise dans 25 ml d'éthanol absolu. On obtient 4,5 g de chlorhydrate de diphényl-3,4 aza-1 bicyclo[2,2,2]octane dont le point de fusion est supérieur à 230°C.

Analyse pour: $C_{19}H_{21}N$, HCl, 1/2 $H_2O$

| | C | H | N | Cl |
|---|---|---|---|---|
| Calculé | 73,8 | 7,45 | 4,52 | 11,5 |
| Trouvé | 72,1 | 7,3 | 4,2 | 11,1 |

*Proprietes Pharmacologiques*

1°) *Action des produits sur l'hypothermie provoquee par la reserpine chez la souris*

L'efficacité des composés de la présente invention comme antidépresseurs a été démontrée à l'aide du test d'hypothermie à la réserpine chez la souris, selon la méthode de D. M. ASKEN, Life Sciences 1963, tome 2, p. 725.

Les résultats sont exprimés par une $DE_{50}$ ou dose de produit, en mg/kg, capable d'exercer un effet antihypothermisant égal à 50% de l'effet antihypothermisant provoque par 15 mg/kg (per os) d'imipramine.

Le tableau ci-après donne, à titre d'exemple, le résultat obtenu avec le composé de l'exemple 4.

| Produit | $DE_{50}$ p.o |
|---|---|
| Exemple 4 | 2 |
| Imipramine | 3 |

2°) *Action des produits sur la recapture des monoamines cerebrales*

On sait que les produits antidépresseurs actuellement connus possèdent la propriété d'inhiber la recapture des monoamines cérébrales.

L'activité comme antidépresseur des produits selon l'invention a donc été démontrée, in vitro, à l'aide du test d'inhibition de la recapture des amines cérébrales par les synaptosomes de cerveau de rat, selon la méthode de KANNENGIESSER et Coll. (Biochem. Pharmacol. *22* 73, 1973).

Les résultats sont exprimés par une concentration inhibitrice $CI_{50}$, qui représente la dose de produit en micromoles par litre diminuant de 50% la recapture de la noradrénaline (NA), de la dopamine (DA), de la sérotonine (5-HT) ou de la choline dans les régions spécifiques du cerveau (hypothalamus, striatum, medulla + pons) où prédominent les neurones correspondants.

Les résultats obtenus avec le produit de l'exemple 4 sont donnés à titre d'exemple dans le tableau suivant. Dans ce tableau sont donnés également, à titre comparatif, les résultats obtenue avec trois antidépresseurs de référence (amphétamine, nomifensine, imipramine).

| | $CI_{50}$ | | | |
|---|---|---|---|---|
| PRODUIT | NA (HYPHOTHALAMUS) | DA (STRIATUM) | 5-HT (MEDULLA + PONS) | CHOLINE (STRIATUM) |
| AMPHETAMINE | 0,012 | 1,7 | 5 | 1000 |
| NOMIFENSINE | 0,022 | 0,65 | 4 | 1000 |
| IMIPRAMINE | 0,13 | 55 | 0,08 | 30 |
| EXEMPLE 4 | 0,045 | 0,60 | 0,30 | 25 |

Le produit de l'exemple 4 est actif à la fois sur les quatre médiateurs utilisés. Son effet inhibiteur se rapproche de celui de l'imipramine en ce qui concerne la NA, la 5-HT et la choline, mais est très supérieur à celui de l'imipramine en ce qui concerne la recapture de la dopamine au niveau du striatum.

Sur ce dernier point le produit de l'exemple 4 se rapproche de la nomifensine, mais il exerce sur la recapture neuronale de la choline une activité inhibitrice que ne possède pas la nomifensine.

Ces deux effets inhibiteurs conjoints sur la recapture de la dopamine et de la choline témoignent des propriétés prodopaminergiques et anticholinergiques centrales des composés de formule (I), propriétés qui sont en liaison avec l'application de ces composés au traitement de la maladie de Parkinson [cf. The Pharmacotherapy of Parkinsonism—R. M. PINDER—Progress. in medicinal chemistry *9*, 191 (1973)].

*Proprietes Toxicologiques*

Les toxicités aiguës des composés de formule (I) ont été déterminées chez la souris mâle $CD_1$ (Charles River) par voie orale. Les $DL_{50}$ ont été calculées, après 3 jours d'observation, par la méthode cumulative de J. J. REED et H. MUENCH. (Amer. J. Hyg. *27*, 493, 1938).

Les composés de formule (I) sont des substances relativement peu toxiques chez la souris. A titre d'exemple la $DL_{50}$ est de 200 mg/kg pour le composé de l'exemple 4.

*Utilisation Therapeutique*

Les composés de formule (I) et leurs sels pharmaceutiquement acceptables peuvent être utilisés en thérapeutique humaine sous forme de comprimés, capsules, gélules, suppositoires, solutions, ingérables ou injectables, etc... pour le traitement des états dépressifs et comme médicaments antiparkinsoniens.

La posologie dépend des effects recherchés et de la voie d'administration utilisée. Par exemple, par voie orale, elle peut être comprise entre 5 et 100 mg de substance active par jour, avec des doses unitaires allant de 1 à 20 mg.

**Revendications**

1. Les composés de formule:

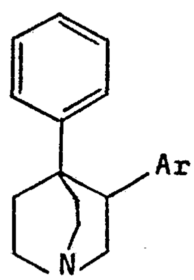

(I)

dans laquelle Ar représente un groupe phényle non substitué ou un groupe phényle substitué par un atome d'halogène ou par un groupe trifluorométhyle, et leurs sels d'addition avec les acides.

2. Composé selon la revendication 1 dans lesquels Ar est un groupe phényle non substitué.

3. Procédé pour la préparation des composés de formule (I) selon la revendication 1, caractérisé en ce que l'on fait réagir la phényl-4 quinuclidinone-3 avec un dérivé organométallique de formule Ar—M, dans laquelle Ar a la même signification que dans la formule (I) et M est un atome de lithium ou un groupe Mg—X, X étant un atome d'halogène, en ce que l'on déshydrate le composé de formule:

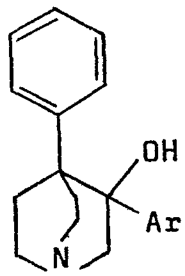

(III)

ainsi obtenu, et en ce que l'on hydrogène catalytiquement le composé de formule:

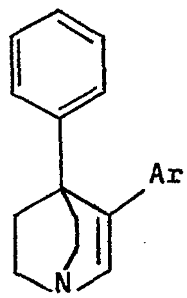

(II)

ainsi obtenu.

4. Médicament pour le traitment des états dépressifs et de la maladie de Parkinson, caractérisé en ce qu'il contient comme preincipe actif un composé de formule (I) selon la revendication 1 ou un sel d'un tel composé avec un acide pharmaceutiquement acceptable.

5. Médicament selon la revendication 4, caractérisé en ce qu'il contient comme principe actif le diphényl-3,4 aza-1 bicyclo[2,2,2]octane ou un sel de ce composé avec un acide pharmaceutiquement acceptable.

6. Les composés de formule:

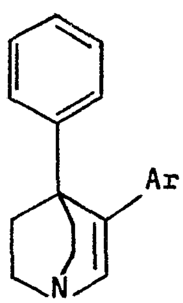

(II)

dans laquelle Ar a les mêmes significations que dans la formule (I) de la revendication 1.

7. Les composés de formule:

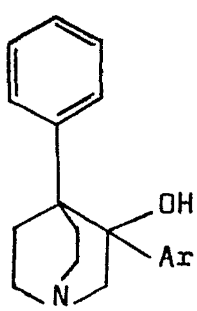

(III)

dans laquelle Ar a les mêmes significations que dans la formule (I) de la revendication 1.

## Claims

1. The compounds of formula:

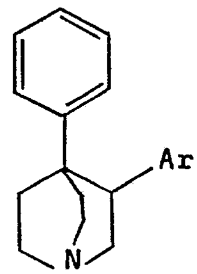

(I)

in which Ar represents an unsubstituted phenyl group or a phenyl group substituted by a halogen atom or by a trifluoromethyl group, and their salts of addition with acids.

2. Compounds according to claim 1 in which Ar is an unsubstituted phenyl group.

3. Process for the preparation of the compounds of formula (I) according to claim 1, characterised in that 4-phenyl-3-quinuclidinone is reacted with an organo-metallic derivative of formula Ar—M, in which Ar has the same significance as in formula (I) and M is a lithium atom or a Mg—X group, X being a halogen atom, in that the compound of formula:

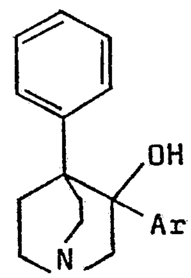

(III)

thus obtained is dehydrated, and in that the compound of formula

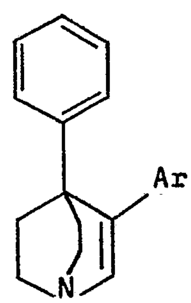

(II)

thus obtained is catalytically hydrogenated.

4. Medicament for the treatment of states of depression and of Parkinson's disease, characterised in that it contains as the active principle a compound of formula (I) according to claim 1 or a salt of such a compound with a pharmaceutically acceptable acid.

5. Medicament according to claim 4, characterised in that it contains as the active principle 3,4-diphenyl-1-aza-[2,2,2]bicyclo-octane or a salt of this compound with a pharmaceutically acceptable acid.

6. The compounds of the formula:

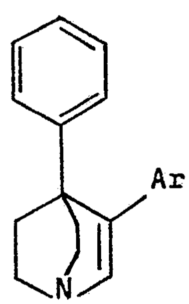

(II)

in which Ar has the same significance as in the formula (I) of claim 1.

7. The compounds of the formula:

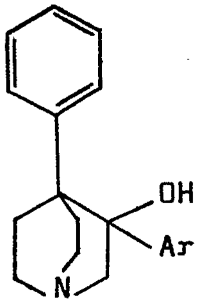

(III)

in which Ar has the same significance as in the formula (I) of claim 1.

**Patentansprüche**

1. Verbindungen der Formel:

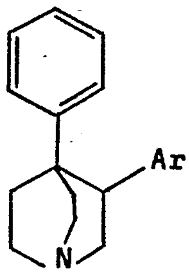

(I)

in der Ar eine nichtsubstituierte Phenylgruppe oder eine durch ein Halogenatom oder eine Trifluoromethylgruppe substituierte Phenylgruppe bedeutet sowie ihre Säureadditionssalze.

2. Verbindungen nach Anspruch 1, in denen Ar eine nichtsubstituierte Phenylgruppe ist.

3. Verfahren zur Herstellung von Verbindungen der Formel (I) gemäß Anspruch 1, dadurch gekennzeichnet, daß man 4-Phenyl-3-chinuclidinon mit einer organometallischen Verbindung der Formel Ar— M zur Umsetzung bringt, in der Ar die gleiche Bedeutung wie in Formel (I) hat und M eine Lithiumatom oder eine Gruppe Mg—X bedeutet, in der X ein Halogenatom ist, daß man die auf diese Weise erhaltene Verbindung der Formel:

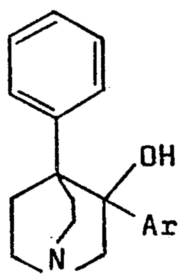

(III)

dehydratisiert und die auf diese Weise erhaltene Verbindung der Formel:

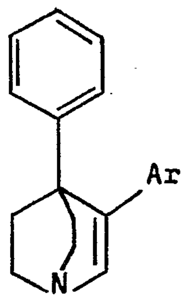

(II)

einer katalytischen Hydrierung unterwirft.

4. Pharmazeutische Zubereitung zur Behandlung depressiver Zustände und der Parkinson'schen

Krankheit, dadurch gekennzeichnet, daß sie als Wirkstoff eine Verbindung der Formel (I) gemäß Anspruch 1 oder ein Salz dieser Verbindung mit einer pharmazeutisch verträglichen Säure enthält.

5. Pharmazeutische Zubereitung nach Anspruch 4, dadurch gekennzeichnet, daß sie als Wirkstoff 3.4-Diphenyl-1-aza-(2.2.2)-bicyclooctan oder ein Salz dieser Verbindung mit einer pharmazeutisch verträglichen Säure enthält.

6. Verbindungen der Formel:

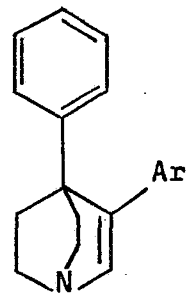

(II)

in der Ar die gleichen Bedeutungen wie in Formel (I) des Anspruchs 1 hat.

7. Verbindungen der Formel:

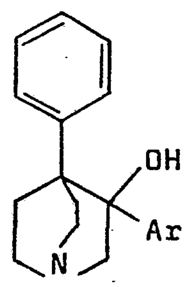

(III)

in der Ar die gleichen Bedeutungen wie in Formel (I) des Anspruchs 1 hat.